# EUROPEAN PATENT APPLICATION

(11) **EP 4 825 418 A1**
(43) Date of publication of application: **21.10.2026**
(21) Application number: 25305574.3
(22) Date of filing: 18.04.2025
(51) Int. Cl.: A61M 5/31, A61M 5/315

(54) **PLUNGER ELEMENT FOR AN INJECTION DEVICE**

(71) Applicant: Becton Dickinson France, 38800 Le Pont-de-Claix (FR)
(72) Inventor: WONG, Yun hui, 538862 Singapore (SG); KUMAR, Jithendra, 731682 Singapore (SG); CHIA Yu Leng, Neville, 120110 Singapore (SG)
(74) Representative: Germain Maureau

(57) **Abstract**

A plunger element (100) including a shaft (120) extending between a proximal end (122) and a distal end (124) along an axis (A-A), at least a portion of the shaft being configured to be received in a barrel (16) of an injection device (100), the shaft including a display surface (126) extending between the proximal end and the distal end of the shaft; and a marking (128) included on the display surface, the marking being configured to indicate a measurement corresponding to a volume of the material contained in the barrel.

## Description

### FIELD

The present disclosure relates to the field of injection devices in medical and/or cosmetic injection procedures. Particularly, the present disclosure relates to a plunger element configured for use on an injection device in a medical and/or injection procedure.

### BACKGROUND

An injection device configured to deliver a material contained in the injection device to a user by injection, such as a medical injection device, a cosmetic injection device, and/or an ear, nose, and throat spray device, typically includes a graduation marking corresponding to a measurement of a volume of the material contained within the injection device.

Currently available injection devices typically include the graduation marking on a barrel of the injection device, which is configured to contain the material. However, when a stopper of the injection device, which is configured to seal the volume of the material within the barrel of the injection device, is in alignment with and/or positioned behind the graduation markings, a color contrast between the graduation markings and the stopper is reduced, resulting in a decreased readability of the graduation markings by a user of the injection device.

Additionally or alternatively, depending on a position of the injection device within a hand of the user, the graduation markings may become inverted and/or out of a line of sight of the user, also resulting in a decreased readability of the graduation markings.

Additionally or alternatively, a grip element of the injection device, which is configured to be mounted to the injection device to improve ergonomics, control, and safety in handling of the injection device, may obstruct a view of the graduation markings when the grip element is mounted to the injection device.

It is desirable to provide a plunger element configured for use on an injection device that is configured to optimize readability of a graduation marking corresponding to a volume of material contained in the injection device. Additionally, it is desirable to provide a grip element configured to be used on an injection device, in combination with the plunger element, to further optimize readability of the graduation marking, while improving ergonomics, control, and safety in handling of the injection device.

### SUMMARY

According to aspects of the disclosure, a plunger element configured for use on an injection device is provided. The injection device includes at least a barrel configured to contain a material. The plunger element includes a shaft extending between a proximal end and a distal end along an axis. At least a portion of the shaft is configured to be received in the barrel of the injection device. The shaft includes a display surface extending between the proximal end and the distal end of the shaft. The plunger element includes a marking included on the display surface. The marking is configured to indicate a measurement corresponding to a volume of the material contained in the barrel.

According to aspects of the disclosure, the display surface may be configured to face the barrel when the shaft is received in the barrel.

According to aspects of the disclosure, the display surface may be included in a first color and the marking may be included in a second color different from the first color of the display surface.

According to aspects of the disclosure, the marking may be in the form of a graduation number configured to indicate the measurement.

According to aspects of the disclosure, the marking may be in the form of a graduation line configured to indicate the measurement.

According to aspects of the disclosure, the shaft may be substantially cylindrical.

According to aspects of the disclosure, the display surface may include a circumference and the marking may be configured to be visible within a range of 180° and 360° about the circumference of the display surface.

According to aspects of the disclosure, an injection device is provided. The injection device includes a barrel configured to contain a material and the plunger element according to any aspect of the disclosure presented herein.

According to aspects of the disclosure, the barrel may include a wall extending between a proximal end and a distal end along the axis, the wall may define a chamber extending between the proximal end and the distal end of the wall, and the chamber may be configured to receive the plunger element.

According to aspects of the disclosure, the wall and the display surface may be configured to define a gap between the wall and the display surface when the plunger element is received within the chamber and the gap may include a distance within a range of 40mm and 90mm.

According to aspects of the disclosure, the injection device may include a grip element including a body extending between a proximal end and a distal end along an axis and the body may be configured to be engaged by a user between the proximal end and the distal end of the body and to surround at least a portion of the barrel between the proximal end and the distal end of the body.

According to aspects of the disclosure, the body may be configured to receive a radial force exerted by the user when the body is engaged by the user.

According to aspects of the disclosure, the body may define an aperture configured to align with the marking.

According to aspects of the disclosure, the body may include a pointer configured to signal to the marking.

According to aspects of the disclosure, a method of determining an amount of material contained within an injection device is provided. The method includes providing the plunger element according to any aspect of the disclosure provided herein and reading the markings included on the display surface.

In the manner described and according to aspects illustrated herein, the plunger element, the injection device, and the method are configured to optimize readability of a graduation marking corresponding to a volume of material contained in an injection device. Additionally, the injection device is configured to allow for optimized readability of the graduation marking, while improving ergonomics, control, and safety in handling of the injection device.

### BRIEF DESCRIPTION OF THE DRAWINGS

Aspects of an example will be described with reference to the drawings, where like numerals represent like parts:
Figure 1 shows a front view of a plunger element configured for use on an injection device according to aspects of the present disclosure;
Figure 2 shows a front view of a grip element configured for use on the injection device according to Figure 1 according to aspects of the disclosure;
Figure 3 shows a front view of the grip element according to Figure 2 according to aspects of the disclosure;
Figure 4 shows a front view of a modified configuration of the grip element according to Figure 2 according to aspects of the disclosure;
Figure 5 shows a rear view of the grip element according to Figure 2 according to aspects of the disclosure;
Figure 6 shows a rear view of a modified configuration the grip element according to Figure 2 according to aspects of the disclosure; and
Figure 7 shows a rear view of a modified configuration of the grip element according to Figure 2 according to aspects of the disclosure.

### DETAILED DESCRIPTION

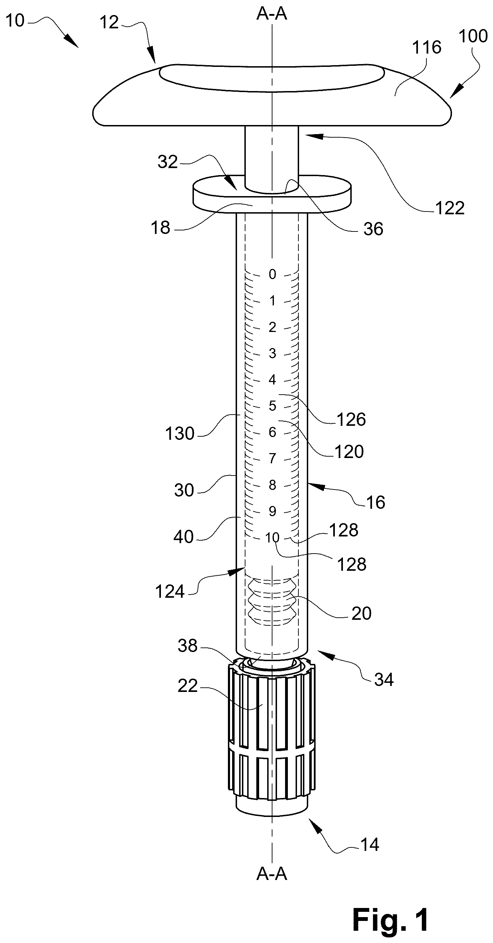

A plunger element configured for use on an injection device according to aspects of the disclosure is described with reference to Figures 1-7. Like numerals represent like parts, and the plunger element will generally be referred to by the reference numeral 100. Although the plunger element 100 is described with reference to specific examples, it should be understood that modifications and changes may be made to these examples without going beyond the general scope as defined by the claims. In particular, individual characteristics of the various examples shown and/or mentioned herein may be combined in additional examples. Consequently, the description and the drawings should be considered in a sense that is illustrative rather than restrictive. The Figures, which are not necessarily to scale, depict illustrative aspects and are not intended to limit the scope of the disclosure. The illustrative aspects depicted are intended only as exemplary.

The term "exemplary" is used in the sense of "example," rather than "ideal." While aspects of the disclosure are amenable to various modifications and alternative forms, specifics thereof have been shown by way of example in the drawings and will be described in detail. It should be understood, however, that the intention is not to limit aspects of the disclosure to a particular example described. On the contrary, the intention of this disclosure is to cover all modifications, equivalents, and alternatives falling within the scope of the disclosure.

Various materials, methods of construction, methods of fastening, and the like may be described in the context of disclosed examples. Those skilled in the art will recognize known substitutes for the materials, construction methods, fastening methods, and the like, all of which are contemplated as compatible with the disclosed example and are intended to be encompassed by the appended claims.

As used in this disclosure and the appended claims, the singular forms "a," "an," and "the" include plural referents, unless the content clearly dictates otherwise. As used in this disclosure and the appended claims, the term "or" is generally employed in a sense including "and/or," unless the content clearly dictates otherwise.

Throughout the description, including the claims, the terms "comprising a," "including a," and "having a" should be understood as being synonymous with "comprising one or more," "including one or more," and "having one or more" unless otherwise stated. In addition, any range set forth in the description, including the claims, should be understood as including its end value(s), unless otherwise stated. Specific values for described elements should be understood to be within accepted manufacturing or industry tolerances known to one of skill in the art, and any use of the terms "substantially," "approximately," and "generally" should be understood to mean falling within such accepted tolerances.

When an element or feature is referred to herein as being "on," "engaged to," "connected to," or "coupled to" another element or feature, it may be directly on, engaged, connected, or coupled to the other element or feature, or intervening elements or features may be present. In contrast, when an element or feature is referred to as being "directly on," "directly engaged to," "directly connected to," or "directly coupled to" another element or feature, there may be no intervening elements or features present. Other words used to describe the relationship between elements or features should be interpreted in a like manner (e.g., "between" versus "directly between," "adjacent" versus "directly adjacent," etc.).

Spatially relative terms, such as "top," "bottom," "middle," "inner," "outer," "beneath," "below," "lower," "above," "upper," and the like may be used herein for ease of description to describe one element or relationship of a feature to another element(s) or feature(s) as illustrated in the drawings. Spatially relative terms may be intended to encompass different orientations of a device in use or operation in addition to the orientation depicted in the drawings. For example, if the device in the drawings is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Although the terms "first," "second," "third," etc. may be used herein to describe various elements, components, regions, layers, sections, and/or parameters, these elements, components, regions, layers, sections, and/or parameters should not be limited by these terms. These terms are only used to distinguish one element, component, region, layer, section, or parameter from another element, component, region, layer, section, or parameter. Thus, a first element, component, region, layer, section, or parameter discussed herein could be termed a second element, component, region, layer, section, or parameter without departing from the teachings of the present disclosure.

As shown by Figures 1-2, the plunger element 100 is configured for use on an injection device 10. Additionally or alternatively, it is contemplated that the plunger element 100 may be referred to as and/or considered to be included by the injection device 10. However, the plunger element 100 will be described herein as being independent of the injection device 10, unless description of the plunger element 100 being included by the injection device 10 is otherwise necessary.

Referring to Figures 1-2, the injection device 10 to which the plunger element 100 is configured for use on may be configured to deliver a material (not shown) contained in the injection device 10 to a user by injecting, spraying, and/or otherwise dispensing a fluid to and/or within a body part of the user and/or a patient of the user during an injection procedure. However, the injection device 10 will be described herein as being configured to deliver the material by injecting a fluid, unless description of the injection device 10 being configured to deliver the material by spraying and/or otherwise dispensing a fluid is otherwise necessary. Accordingly, it is contemplated that the injection device 10 may be configured to be, referred to as, and/or considered to be a "syringe, a "medical device," and/or a "cosmetic device." It is contemplated that the material contained within the injection device 10 may be a fluid having one or more of medicinal and cosmetic properties.

In examples, the injection device 10 extends between a proximal end 12 and a distal end 14 along an axis A-A. Additionally or alternatively, the axis A-A of the injection device 10 may be referred to as and/or considered to be the axis A-A of the plunger element 100. Additionally or alternatively, the axis A-A of the injection device 10 may be referred to as and/or considered to be the axis A-A of each component configured for use on and/or included by the injection device 10 (e.g. a barrel 16 included by the injection device 10, a grip element 200 (discussed in further detail below) configured for use on the injection device 10, and/or the like). In examples, the axis A-A may extend substantially longitudinally. Accordingly, the axis A-A may be referred to as and/or considered to be a "longitudinal axis."

It is contemplated that the terms "proximal" and "distal" as used herein may be understood as a position and/or location corresponding to a component, device, and/or a portion thereof in relation to a hand (not shown) of the user when the user is holding the plunger element 100, the grip element 200, and/or the injection device 10. In examples, the term "proximal," with respect to the plunger element 100, the grip element 200, and/or the injection device 10, may correspond to a position and/or location provided at a distance closer to the hand of the user holding the plunger element 100, the grip element 200, and/or the injection device 10 than a "distal" position and/or location. The term "distal," with respect to the plunger element 100, the grip element 200, and/or the injection device 10, may correspond to a position and/or location provided at a distance that is farther from the hand of the user holding the plunger element 100, the grip element 200, and/or the injection device 10 than a "proximal" position and/or location. Additionally or alternatively, a "distal direction" may be understood as a direction of delivery of the material from the injection device 10 during performance of the injection procedure and a "proximal direction" may be understood as a direction substantially opposite the direction of delivery of the material from the injection device 10 and/or a direction toward the hand of the user holding the plunger element 100, the grip element 200, and/or injection device 10.

In examples, the injection device 10 may include a barrel 16 (may also be referred to as and/or considered to be a "reservoir") configured to contain the material; a finger support 18 extending radially outward from the barrel 16 and configured to allow the user to handle and control the injection device 10; the grip element 200, which is configured to improve ergonomics, control, and safety in the handling of the injection device 10; the plunger element 100, which is configured to be received within the barrel 16 to decrease a volume within the barrel 16 to push the material out of the barrel 16 and to increase a volume within the barrel 16 to draw the material into the barrel 16 and/or aspirate the injection device 10; a stopper 20 (may also be referred to as and/or considered to be a "piston") configured to be affixed to the plunger element 100 and to seal the volume and/or the barrel 16; a tip (may also be referred to as and/or considered to be a "needle" or a "nozzle") (not shown) configured to deliver the material; a hub (not shown) configured to connect the tip to the barrel 16; and a cap element 22 (may also be referred to herein as a "shield") configured to protect and/or seal the tip. In examples, one or more of the barrel 16, the finger support 18, and the hub may be formed from a glass material and/or the like.

It is contemplated that the terms "outer," "outward," "inner," and "inward" may be understood in relation to the axis A-A, such that the terms "outer" and "outward" may correspond to a position and/or direction farther from and/or away from the axis A-A in contrast with an "inner" and "inward" position and/or direction and the terms "inner" and "inward" may correspond to a position and/or direction closer to and/or toward the axis A-A in contrast with an "outer" and "outward" position and/or direction.

Referring to Figures 1-2, the barrel 16 includes a wall 30 (may also be referred to as and/or considered to be a "sidewall") extending substantially axially between a proximal end 32 and a distal end 34. It is contemplated that the proximal end 32 and the distal end 34 of the wall 30 may be referred to as and/or considered to be the proximal end 32 and the distal end 34 of the barrel 16. In examples, the wall 30 defines a first opening 36 at or adjacent to the proximal end 32 of the wall 30 and a second opening 38 at or adjacent to the distal end 34 of the wall 30. Additionally, the wall 30 defines a chamber 40 extending substantially axially between the first opening 36 and the second opening 38 of the wall 30. The chamber 40 is configured to be in communication with the first opening 36 and the second opening 38 of the wall 30. In examples, the barrel 16 is configured to receive the plunger element 100. In particular, the barrel 16 is configured to receive the plunger element 100 through the first opening 36 and the chamber 40 defined by the wall 30. In examples, the wall 30 is substantially transparent. In examples, the wall 30 is substantially cylindrical. Additionally or alternatively, the wall 30 includes a circumference.

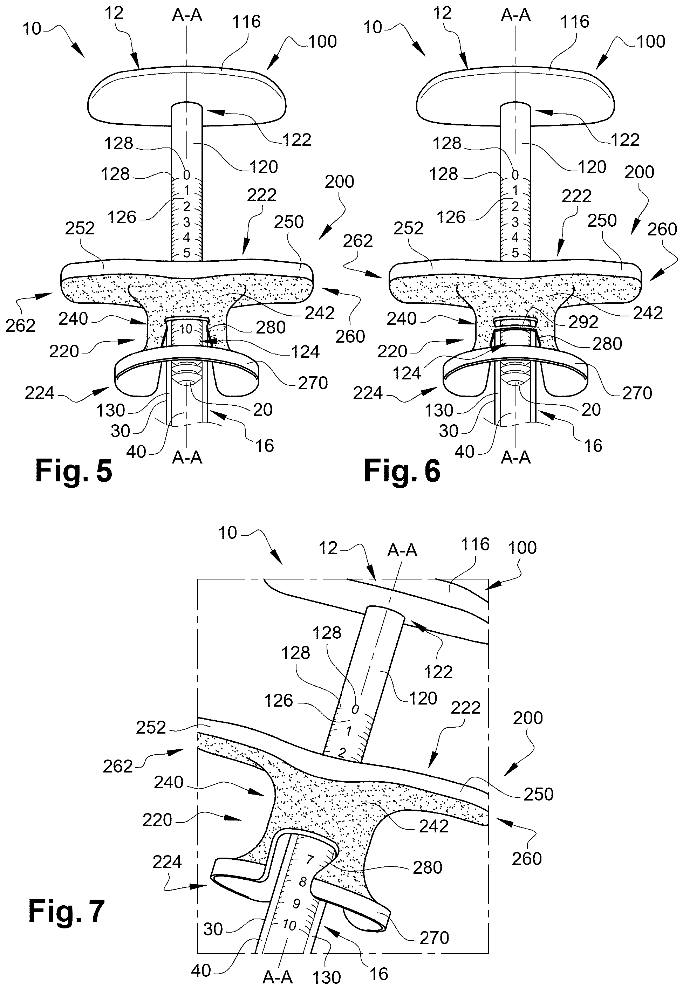

As shown by Figures 1-2 and 5-7, the plunger element 100 includes a shaft 120 extending substantially axially between a proximal end 122 and a distal end 124. It is contemplated that the proximal end 122 of the shaft 120 may correspond to the proximal end 12 of the injection device 10. At least a portion of the shaft 120 is configured to be received in the barrel 16. In particular, at least a portion of the shaft 120 is configured to be received in the chamber 40 of the barrel 16. In examples, the plunger element 100 is configured to reciprocate between a first position (see Figures 5-6) and a second position (see Figures 1-2) in the chamber 40. In the first position of the plunger element 100, the distal end 124 of the shaft 120 is closer to the proximal end 32 of the wall 30 of the barrel 16 than the distal end 124 of the shaft 120 in the second position of the plunger element 100. In the second position of the plunger element 100, the distal end 124 of the shaft 120 is farther from the proximal end 32 of the wall 30 and/or closer to the distal end 34 of the wall 30 than the distal end 124 of the shaft 120 in the first position of the plunger element 100.

In examples, the plunger element 100 may include a handle member 132 oriented at or adjacent to the proximal end 122 of the shaft 120. The handle member 132 may be configured to be pushed by the user to transition the plunger element 100 from the first position of the plunger element 100 toward and/or to the second position of the plunger element 100 and to be pulled by the user to transition the plunger element 100 from the second position of the plunger element 100 toward and/or to the first position of the plunger element 100.

In examples, the shaft 120 includes a display surface 126 extending between the proximal end 122 and the distal end 124 of the shaft 120. The display surface 126 is configured to communicate information to the user. Accordingly, the display surface 126 is configured to face radially outward from the shaft 120. Additionally or alternatively, the display surface 126 is configured to face the wall 30 of the barrel 16 when the shaft 120 is received in the barrel 16. In examples, the shaft 120 and, thus, the display surface 126, are substantially cylindrical. Additionally or alternatively, the shaft 120 and, thus, the display surface 126, include a circumference. Including the shaft 120 and, thus, the display surface 126, to be substantially cylindrical and/or to include the circumference allows the display surface 126 to communicate information to the user dynamically, at a plurality of angles, so as to allow the display surface 126 to be readable regardless of a position in which the injection device 10 is handled by the user. In examples, the display surface 126 is configured to extend and/or to communicate information to the user within a range of 180° and 360° about the circumference of the display surface 126.

The plunger element 100 includes a marking 128 (may also be referred to herein as a "graduation marking" and/or a "scale") included on the display surface 126 of the shaft 120. In examples, the marking 128 is configured to indicate a measurement corresponding to a volume of the material contained within the barrel 16. In examples, the plunger element 100 may include a plurality of the marking 128 configured to indicate a plurality of measurements corresponding a volume of the material contained within the barrel 16. However, the plurality of the marking 128 will be referred to herein as "the marking," unless specific reference to the plurality of the marking 128 is otherwise necessary.

In examples, the marking 128 may be in the form of a graduation number (may also be referred to herein as a "numerical digit") configured to indicate the measurement. In examples in which the plunger element 100 includes a plurality of the marking 128, the graduation numbers may increase and/or be included in ascending order from the proximal end 122 of the shaft 120 toward the distal end 124 of the shaft. In this manner, a corresponding marking 128 signaled by a pointer 42, 290, 292 (discussed in further detail below) included by one or more of the barrel 16 and the grip element 200 decreases as a volume of the material contained within the barrel 16 decreases when the plunger element 100 is transitioned from the first position of the plunger element 100 toward the second position of the plunger element 100.

Additionally or alternatively, the marking 128 may be in the form of a graduation line configured to indicate the measurement. In examples, the graduation line of the marking 128 may correspond to the graduation number of the marking 128. Additionally or alternatively, the graduation line of the marking 128 may be in the form of a major graduation line corresponding to the graduation number of the marking 128 and a minor graduation line corresponding to an increment between a pair of graduation numbers. The marking 128 may be included in graduations (may also be referred to as and/or considered to be "intervals"). In examples, the markings 128 may be included in graduations within a range of 0.01mL and 0.25mL. Additionally or alternatively, the marking 128 may be configured to indicate a description and/or data corresponding to the material contained within the barrel 16, such as a name corresponding to the material, a date corresponding to the material, a usage corresponding to the material, and/or the like.

In examples, the marking 128 included on the display surface 126 of the shaft 120 may be configured to contrast with the display surface 126. Accordingly, the display surface 126 may be included in a first color and the marking 128 may be included in a second color. The second color of the marking 128 may be different from the first color of the display surface 126. In examples, the second color of the marking 128 may be configured to have an increased level of contrast with the first color of the display surface 126. In examples, the second color of the marking 128 may be a hue of black and/or the like and the first color of the display surface 126 may be a hue of white and/or the like.

In examples, the display surface 126 of the shaft 120 and the wall 30 of the barrel 16 are configured to define a gap 130 between the display surface 126 and the wall 30. The distance of the gap 130 may be included within a range of 40mm and 90mm and, particularly, within a range of 50mm and 80mm. In this manner, the gap 130 is configured to improve magnification of the display surface 126 and, thus, the marking 128, through refraction of light through the wall 30. Additionally, in this manner, the gap 130 is configured to reduce friction between the display surface 126 and the wall 30 to enhance transition of the plunger element 100 between the first position of the plunger element 100 and the second position of the plunger element 100.

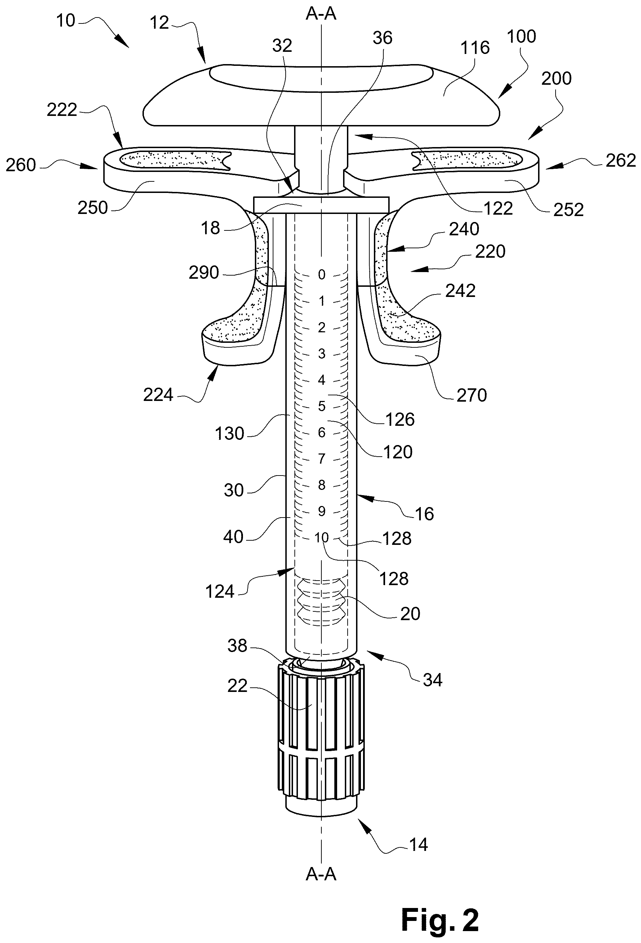

As shown by Figures 2-7, a grip element 200 may be configured to be mounted to the injection device 10. Additionally or alternatively, it is contemplated that the grip element 200 may be referred to as and/or considered to be included by the injection device 10. However, the grip element 200 will be described herein as being independent of the injection device 10, unless description of the grip element 200 being included by the injection device 10 is otherwise necessary.

Referring to Figures 2-4, the grip element 200 includes a body 220 extending substantially axially between a proximal end 222 and a distal end 224. It is contemplated that the proximal end 222 and the distal end 224 of the body 220 may be referred to as and/or considered to be the proximal end 222 and the distal end 224 of the grip element 200. The body 220 includes an outer surface 226 and an inner surface 228 extending between the proximal end 222 and the distal end 224 of the body 220.

Referring to Figures 2-4, the inner surface 228 of the body 220 is configured to receive the injection device 10 such that the injection device 10 may be inserted into the body 220. Additionally or alternatively, the inner surface 228 of the body 220 is configured to engage the injection device 10. In examples, the inner surface 228 of the body 220 may include a coupling member (not shown) configured to engage the injection device 10 through an interference-fit connection, a snap-fit connection, and/or the like. Accordingly, the grip element 200 is configured to be mounted to the injection device 10.

Additionally or alternatively, it is contemplated that the grip element 200 may be formed on and/or included integrally on the injection device 10. Additionally or alternatively, it is contemplated that the grip element 200 may be configured to be, referred to as, and/or considered to be the finger support 18 of the injection device 10, such that the grip element 200 is configured to replace and/or be interchangeable with a corresponding finger support 18 of a corresponding injection device 10. However, the grip element 200 will be described herein as being configured to engage the injection device 10, unless description of the grip element 200 being configured to be formed on and/or included integrally on the injection device 10 is otherwise necessary.

In examples, the inner surface 228 of the body 220 defines a first opening 230 at or adjacent to the proximal end 222 of the body 220 and a second opening 232 at or adjacent to the distal end 224 of the body 220. The first opening 230 and the second opening 232 of the body 220 are oriented about the axis A-A. Additionally, the inner surface 228 of the body 220 defines a cavity 234 extending substantially axially between the first opening 230 and the second opening 232 of the body 220. The cavity 234 of the body 220 is configured to be in communication with the first opening 230 and the second opening 232 of the body 220. Additionally or alternatively, it is contemplated that the first opening 230 and the second opening 232 of the body 220 may be configured to be, referred to as, and/or considered to be included by the cavity 234.

Each of the first opening 230, the second opening 232, and the cavity 234 of the body 220 are configured to receive at least a portion of the injection device 10. In examples, the first opening 230 of the body 220 may be configured and/or shaped to receive and/or engage at least a portion of the plunger element 100 when the injection device 10 is inserted into the body 220. Additionally, the second opening 232 may be configured and/or shaped to receive and/or engage at least a portion of the barrel 16 of the injection device 10 when the injection device 10 is inserted into the body 220. Additionally, the cavity 234 may be configured and/or shaped to receive and/or engage at least a portion of one or more of the barrel 16 of the injection device 10, the finger support 18 of the injection device 10, and the plunger element 100. **In** examples, the cavity 234 may include one or more of a first section 234a configured and/or shaped to receive and/or engage at least a portion of the barrel 16 of the injection device 10, a second section 234b configured and/or shaped to receive and/or engage at least a portion of the finger support 18 of the injection device 10, and a third section 234c configured and/or shaped to receive and/or engage at least a portion of the plunger element 100.

Each of the first opening 230, the second opening 232, and the cavity 234 of the body 220 may extend radially through and/or open to a clearance 236 at a first face 238 (may also be referred to herein as a "front" and/or a "front face") of the body 220. It is contemplated that the term "face" may be understood in relation to the axis A-A, such that the term "face" may correspond to a position and/or direction perpendicular to the axis A-A. Accordingly, each of the first opening 230, the second opening 232, and the cavity 234 of the body 220 may be configured to receive at least a portion of the injection device 10 through the clearance 236 at the first face 238 of the body 220, so that the injection device 10 may be inserted into the grip element 200 through the first face 238 of the body 220. Additionally or alternatively, each of the first opening 230, the second opening 232, and the cavity 234 of the body 220 may be configured to receive the injection device 10 through one or more of the first opening 230 and the second opening 232 of the body 220, such that the injection device 10 may be inserted into the grip element 200 through one or more of proximal end 222 and the distal end 224 of the body 220. However, the grip element 200 will be described herein as being configured to receive the injection device 10 through the first face 238 of the body 220, unless description of the grip element 200 being configured to receive the injection device 10 through one or more of the proximal end 222 and the distal end 224 of the body 220 is otherwise necessary.

Referring to Figure 2, the clearance 236 is configured to allow the user to view the display surface 126 of the shaft 120 and, thus, the marking 128 included on the display surface 126. In particular, the clearance 236 is configured to align with the marking 128. Including the clearance 236 at the first face 238 of the body 220 allows the clearance 236 to align with the marking 128 during the injection procedure. In this manner, the clearance 236 is configured to further optimize readability of the marking 128, while improving ergonomics, control, and safety in handling of the injection device 10.

Referring to Figures 5-7, the body 220 may define an aperture 280 (may also be referred to as and/or considered to be a "window") configured to allow the user to view the display surface 126 of the shaft 120 and, thus, the marking 128 included on the display surface 126. In particular, the aperture 280 may be configured to align with the marking 128. In examples, the aperture 280 may be defined at a second face 239 (may also be referred to herein as a "rear" and/or a "rear face") of the body 220. In examples, the second face 239 of the body 220 may be substantially opposite the first face 238 of the body 220. Including the aperture 280 at the second face 239 of the body 220 allows the aperture 280 to align with the marking 128 during the injection procedure. Additionally, including the aperture 280 at the second face 239 of the body 220, in addition to including the clearance 238 provided at the first face 238 of the body 220, allows the display surface 126 to communicate information to the user dynamically, at a plurality of angles, so as to allow the display surface 126 to be readable regardless of a position in which the injection device 10 is handled by the user. Referring to Figures 5-6, the aperture 280 may be defined between the proximal end 222 and the distal end 224 of the body 220. Additionally or alternatively, referring to Figure 7, the aperture 280 may extend through the distal end 224 of the body 220 to further remove obstruction caused by the body 220. In this manner, the aperture 280 is configured to further optimize readability of the marking 128, while also improving ergonomics, control, and safety in handling of the injection device 10.

Referring to Figures 1-2 and 5-6, in examples, one or more of the wall 30 of the barrel 16 and the outer surface 226 of the body 220 may include a pointer 42, 290, 292 configured to signal to and/or identify the marking 128 included on the display surface 126 of the shaft 120. In particular, the pointer 42, 290, 292 is configured to signal and/or identify the marking 128 of the plurality of markings 128 corresponding to a volume and/or remaining volume of the material contained in the barrel 16. In examples, the pointer 42, 290, 292 may be in the form of a line, an arrow, an indent, and/or the like that is oriented at or adjacent to the marking 128. Additionally or alternatively, the pointer 42, 290, 292 may be included in a color different from one or more of the first color of the display surface 126 and the second color of the marking 128, thereby increasing a level of contrast between the pointer 42, 290, 292 and the display surface 126 and the marking 128.

Referring to Figure 1, in examples, the wall 30 may include a pointer 42 oriented to overlay the marking 128. The pointer 42 of the wall 30 may be oriented at or adjacent to the proximal end 32 of the wall 30. Referring to Figure 2, additionally or alternatively, the outer surface 226 of the body 220 may include a pointer 290 oriented at the first face 238 of the body 220. The pointer 290 included at the first face 238 of the body 220 may be oriented at or adjacent to the clearance 236 of the body 220. Referring to Figures 5-6, additionally or alternatively, the outer surface 226 of the body 220 may include a pointer 292 oriented at the second face 239 of the body 220. The pointer 292 included at the second face 239 of the body 220 may be oriented at or adjacent to the aperture 280 of the body 220. Referring to Figure 6, in examples, the pointer 292 may extend across the aperture 280 of the body 220. In this manner, readability of the marking 128 may be further optimized.

When the grip element 200 is mounted to the injection device 10 and/or the injection device 10 is inserted into the grip element 200, at least a portion of the plunger element 100 of the injection device 10 extends beyond the proximal end 222 of the body 220 and at least a portion of the barrel 16 of the injection device 10 and the tip of the injection device 10 extend beyond the distal end 224 of the body 220. In this manner, the plunger element 100 of the injection device 10 may be handled by the user beyond the proximal end 222 of the body 220 and the tip of the injection device 10 may be used to deliver the material.

Referring to Figures 2-4, the outer surface 226 of the body 220 is configured to be engaged by the user. In particular, the outer surface 226 of the body 220 is configured to be engaged by the user between the proximal end 222 and the distal end 224 of the body 220. In examples, the outer surface 226 of the body 220 is configured to be engaged by at least a portion of one or more finger (not shown) of a hand of the user and/or a palm (not shown) of the hand of the user. In examples, the outer surface 226 of the body 220 may be configured to be engaged by one or more of a first finger of the hand of the user (may also be referred to as and/or considered to be an "index finger"), a second finger of the hand of the user (may also be referred to as and/or considered to be a "middle finger"), a third finger of the hand of the user (may also be referred to as and/or considered to be a "ring finger"), and the palm of the hand of the user. It is contemplated that the first finger of the hand of the user is oriented adjacent to the second finger of the hand of the user and that the second finger of the hand of the user is oriented adjacent to the third finger of the hand of the user. Additionally or alternatively, it is contemplated that the second finger of the hand of the user is oriented between the first finger and the third finger of the hand of the user.

The outer surface 226 of the body 220 is configured to receive a radial force exerted by the user when the body 220 is engaged by the user. In examples, the body 220 is configured to extend between one or more of the first finger and the second finger of the hand of the user and the second finger and the third finger of the hand of the user. In examples, the body 220 is elongated between the proximal end 222 and the distal end 224 of the body 220. The body 220 may include an elongated central portion 240 extending between the proximal end 222 and the distal end 224 of the body 220. Additionally or alternatively, the body 220 may be substantially tubular in shape. In examples, the body 220 extends to a length within a range of 15mm and 40mm and, in particular, within a range of 18mm and 32mm.

In this manner, the body 220 is configured to facilitate a first position of the grip element 200 (may also be referred to as and/or considered to be a "rest position") during the injection procedure. In the first position of the grip element 200, the body 220 is configured to allow the user to rest the grip element 200 between one or more of the first finger and the second finger and the second finger and the third finger of the hand of the user. Additionally or alternatively, in this manner, the body 220 is configured to facilitate a second position of the grip element 200 (may also be referred to as and/or considered to be an "aspiration position") during the injection procedure. In the second position of the grip element 200, the body 220 is configured to allow the user to exert a radial force on and/or to squeeze the grip element 200 between one or more of the first finger and the second finger and the second finger and the third finger of the hand of the user, thereby allowing the user to pull the plunger element 100 in the proximal direction. Accordingly, in this manner, the grip element 200 is configured to improve ergonomics, control, and safety in handling of the injection device 10

In examples, the outer surface 226 of the body 220 may include a pad 242 configured to be engaged by the user. The pad 242 may extend between the proximal end 222 and the distal end 224 of the body 220 and/or throughout the body 220 toward and/or to the proximal end 222 and the distal end 224 of the body 220. The pad 242 may extend circumferentially about and/or throughout a circumference of the body 220. In examples, the body 220 may be formed from a first material and the pad 242 may be formed of a second material. The first material of the body 220 may include a first rigidity and the pad 242 of the body 220 may include a second rigidity. The first rigidity of the first material may be greater than the second rigidity of the second material. In examples, the body 220 may be formed from a durable material, such as a plastic material, a metal material, and/or the like. In examples, the pad 242 may be formed from an elastically deformable material, such as a rubber material, a silicone material, and/or the like. In this manner, the pad 242 is configured to enhance the improved ergonomics, control, and safety of the grip element 200.

Referring to Figures 2-4, the outer surface 226 of the body 220 is configured to receive an axial force exerted by the user when the body 220 is engaged by the user. The grip element 200 includes one or more flange 250, 252 extending radially outward from the proximal end 222 of the body 220. The flange 250, 252 is configured to be engaged by the user to receive an axial force exerted by the user on the outer surface 226 of the body 220. In examples, the flange 250, 252 includes a proximal surface 254 and a distal surface 256. The proximal surface 254 of the flange 250, 252 may be configured to receive an axial force exerted by the user in the distal direction. The distal surface 256 of the flange 250, 252 may be configured to receive an axial force exerted by the user in the proximal direction. It is contemplated that the flange 250, 252 may be configured to be, referred to as, and/or considered to be included by the body 220. Accordingly, the outer surface 226 of the body 220 extends to and/or throughout the flange 250, 252. Additionally or alternatively, the outer surface 226 of the body 220 may be referred to as and/or considered to be the outer surface 226 of the flange 250, 252.

In examples, the grip element 200 includes at least a first flange 250 included on a first side 260 of the body 220 and a second flange 252 included on a second side 262 of the body 220. It is contemplated that the term "side" may be understood in relation to the axis A-A, such that the term "side" may correspond to a position and/or direction perpendicular to the axis A-A. In examples, the first side 260 of the body 220 opposes the second side 262 of the body 220 about the axis A-A. Additionally or alternatively, the first face 238 of the body 220 extends between the first side 260 and the second side 262 of the body 220. Accordingly, the body 220 may be substantially T-shaped.

The distal surface 256 of the first flange 250 may be configured to be engaged by the first finger of the hand of the user and the distal surface 256 of the second flange 252 may be configured to be engaged by the second finger of the hand of the user. Additionally or alternatively, the distal surface 256 of the first flange 250 may be configured to be engaged by the second finger of the hand of the user and the distal surface 256 of the second flange 252 may be configured to be engaged by the third finger of the hand of the user. The proximal surface 254 of one or more of the first flange 250 and the second flange 252 may be configured to be engaged by the palm of the hand of the user. In this manner, the first flange 250 and the second flange 252 are configured to facilitate a third position of the grip element 200 (may also be referred to as and/or considered to be an "injection position") during the injection procedure. In the third position of the grip element 200, the first flange 250 and the second flange 42 are configured to allow the user to exert an axial force and/or to pull the first flange 250 and the second flange 252, thereby allowing the user to push the plunger element 100 in the distal direction. Accordingly, in this manner the grip element 200 is configured to improve ergonomics, control, and safety of the injection device 10.

Referring to Figures 2-4, the grip element 200 may include a rim 270 extending radially outward from the distal end 224 of the body 220. Accordingly, the grip element 200 may be configured to receive one or more of the first finger, the second finger, and the third finger of the hand of the user between the first flange 250 and the second flange 252 and the rim 270. The rim 270 may be configured to be engaged by the user to receive an axial force exerted by the user on the outer surface 226 of the body 220. The rim 270 may be configured to receive an axial force exerted by the user in the distal direction. In particular, the rim 270 may be configured to receive an axial force exerted by one or more of the first finger and the second finger of the hand of the user and the second finger and the third finger of the hand of the user. It is contemplated that the rim 270 may be configured to be, referred to as, and/or considered to be included by the body 220. Accordingly, the outer surface 226 of the body 220 may extend to and/or throughout the rim 270. Additionally or alternatively, the outer surface 226 of the body 220 may be referred to as and/or considered to be the outer surface 226 of the rim 270. In this manner, the body 220 is configured to enhance the improved ergonomics, control, and safety of the injection device 10.

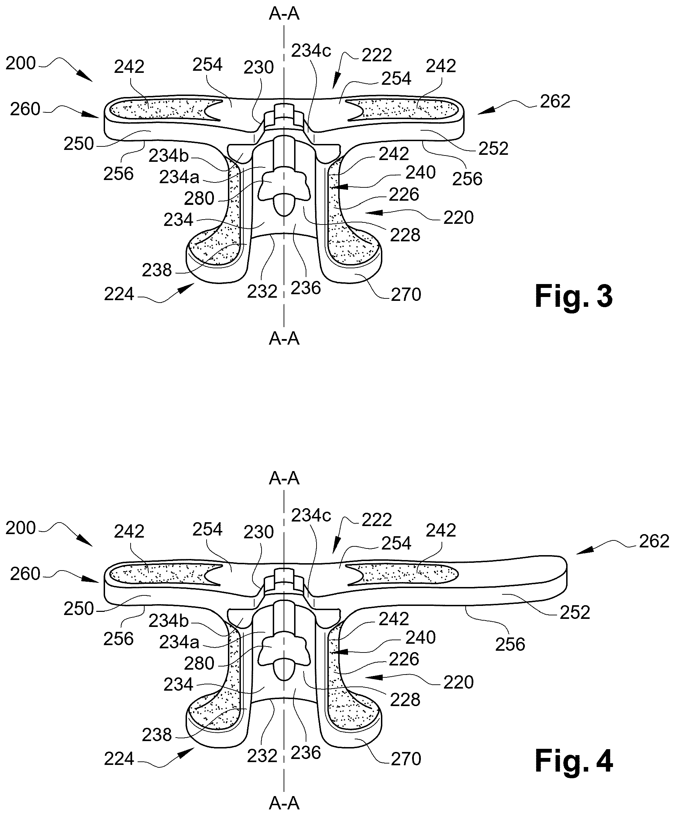

Referring to Figures 3-4, the pad 242 may extend to, throughout, and/or be included on the distal surface 256 of the first flange 250 and the distal surface 256 of the second flange 252. Additionally or alternatively, the pad 242 of the body 220 may extend to, throughout, and/or be included on the proximal surface 254 of the first flange 250 and the proximal surface 254 of the second flange 252. Additionally or alternatively, the pad 242 may extend to, throughout, and/or be included on the rim 270.

In examples, the first flange 250 may extend to a first length and the second flange 252 may extend to a second length. Referring to Figure 3 in examples, the first length of the first flange 250 may be substantially equal to the second length of the second flange 252. In examples, the first length of the first flange 250 and the second length of the second flange 252 may be within a range of 5mm and 40mm and, in particular, within a range of 12mm and 17mm. Alternatively, referring to Figure 4, in examples, the second length of the second flange 252 may be greater than the first length of the first flange 250. In examples, the first length of the first flange 250 may be within a range of 5mm and 20mm and, in particular, within a range of 12mm and 17mm. The second length of the second flange 252 may be within a range of 20mm and 40mm and, in particular, within a range of 23mm and 32mm. The second length of the second flange 252 being greater than the first length of the first flange 250 allows for a greater surface area and, thus, a greater point of contact, for engagement of one or more of the proximal surface 254 and the distal surface 256 of the second flange 252. Accordingly, the distal surface 256 of the second flange 252 may be configured to be engaged by the second finger and the third finger of the hand of the user during the injection procedure. Additionally or alternatively, the proximal surface 254 of the second flange may be configured to be engaged by a larger portion of the palm of the hand of the user during the injection procedure. Alternatively, in examples, it is contemplated that the first length of the first flange 250 may be greater than the second length of the second flange 252, as described in relation to the second length of the second flange 252 being greater than the first length of the first flange 250.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

Additionally, all of the disclosed features of an apparatus may be transposed, alone or in combination, to a method and vice versa.

## Claims

1. A plunger element (100) configured for use on an injection device (10), the injection device including at least a barrel (16) configured to contain a material, the plunger element comprising:
a shaft (120) extending between a proximal end (122) and a distal end (124) along an axis (A-A), at least a portion of the shaft being configured to be received in the barrel of the injection device, the shaft including a display surface (126) extending between the proximal end and the distal end of the shaft; and
a marking (128) included on the display surface, the marking being configured to indicate a measurement corresponding to a volume of the material contained in the barrel.

2. The plunger element (100) according to claim 1, wherein the display surface (126) is configured to face the barrel (16) when the shaft (120) is received in the barrel.

3. The plunger element (100) according to any of claims 1-2, wherein the display surface (126) is included in a first color and the marking (128) is included in a second color different from the first color of the display surface.

4. The plunger element (100) according to any of claims 1-3, wherein the marking (128) is in the form of a graduation number configured to indicate the measurement.

5. The plunger element (100) according to any of claims 1-4, wherein the marking (128) is in the form of a graduation line configured to indicate the measurement.

6. The plunger element (100) according to any of claims 1-5, wherein the shaft (120) is substantially cylindrical.

7. The plunger element (100) according to any of claims 1-6, wherein the display surface (126) includes a circumference and the marking (128) is configured to be visible within a range of 180° and 360° about the circumference of the display surface.

8. An injection device (10) comprising:
a barrel (16) configured to contain a material; and
the plunger element (100) according to any of claims 1-7.

9. The injection device (10) according to claim 8, wherein the barrel (16) includes a wall (30) extending between a proximal end (32) and a distal end (34) along the axis (A-A), the wall defining a chamber (40) extending between the proximal end and the distal end of the wall, and the chamber being configured to receive the plunger element (100).

10. The injection device (10) according to claim 9, wherein the wall (30) and the display surface (126) are configured to define a gap (130) between the wall and the display surface when the plunger element (100) is received within the chamber (40) and the gap may include a distance within a range of 40mm and 90mm.

11. The injection device (10) according to any of claims 8-10, comprising a grip element (200) including a body (220) extending between a proximal end (222) and a distal end (224) along an axis (A-A), the body being configured to be engaged by a user between the proximal end and the distal end of the body and to surround at least a portion of the barrel (16) between the proximal end and the distal end of the body.

12. The injection device (10) according to claim 11, wherein the body (220) is configured to receive a radial force exerted by the user when the body is engaged by the user.

13. The injection device (10) according to any of claims 11-12, wherein the body (220) defines an aperture (280) configured to align with the marking (128).

14. The injection device (10) according to claim any of claims 11-13, wherein the body (220) includes a pointer configured to signal to the marking (128).

15. A method of determining an amount of material contained within an injection device (10), the method comprising:
providing the plunger element (100) according to any of claims 1-7; and
reading the marking (128) included on the display surface (126).
